# EUROPEAN PATENT SPECIFICATION

(11) **EP 3 197 379 B1**
(45) Date of publication and mention of the grant of the patent: **24.10.2018**
(21) Application number: 15766484.8
(22) Date of filing: 18.09.2015
(51) Int. Cl.: A61B 17/64

(54) **DEVICE FOR REPOSITIONING BONE FRACTURE FRAGMENTS**
VORRICHTUNG ZUM REPOSITIONIEREN VON KNOCHENBRUCHFRAGMENTEN
DISPOSITIF DE REPOSITIONNEMENT DE FRAGMENTS DE FRACTURE OSSEUSE

(30) Priority: 22.09.2014 DE 102014113658
(43) Date of publication of application: 02.08.2017
(73) Proprietor: Maquet GmbH, 76437 Rastatt (DE)
(72) Inventor: GÖCKERITZ, Stefan, 99096 Erfurt (DE); KRICKEBERG, Thomas, 76307 Karlsbad (DE); KATZENSTEIN, Bernhard, 76473 Iffezheim (DE)
(74) Representative: Zacco GmbH
(86) International application number: PCT/EP2015/071489
(87) International publication number: WO 2016/046089

(56) References cited:
- EP-A1- 2 436 324
- WO-A1-2010/138715
- WO-A2-2006/126167
- US-A- 5 827 283

## Description

The invention relates to a device for repositioning bone fracture fragments which comprises a carrier unit, a first arm attached to the carrier unit for holding a first bone fracture fragment and a second arm attached to the carrier unit for holding a second bone fracture fragment.
Fractures of the human extremities are usually due to accidents. The repositioning and stabilisation of the fragments are therefore usually unplanned surgical procedures. If the patient's condition, particular the patient's circulation, is stable the operator will treat the fracture without making use of various aids. Generally, depending on the fracture and number of fragments different osteosynthesis material(s) and method(s) are used. Both plates and intramedullary nails are commonly used. So that the intramedullary nail or the plate can be correctly applied, the fragments have to be aligned and/or repositioned with regard to each other. The quality of the alignment is crucial for the treatment result and cannot be changed postoperatively. For correction purposes the only option is a further medical procedure. As the bone fragments always lie inside the soft tissue of the extremities, they can only be accurately repositioned with the help of X-ray image intensifiers. An additional difficulty is the fact that the muscles surrounding the bone contract so that the fragments end up in an unnatural alignment.

Document US 5,827,283 describes a device for locating in a desired position two or more bones or bone pieces that are in an undesired relative position, by bringing such pieces from the undesired position into the desired position. The device comprises a straightforward and simple structure including two arms to be fixed to the jaw members and connected to arm driving motors. In order to correctly align the fragments, forces have to be applied to the fragments from outside. These must be kept constant for the duration of screwing the plates or inserting the intramedullary nails.
Imprecise alignment of the fragments results in rather large rotative misorientation of the fragments with regard to each other which can no longer be changed postoperatively. Due to the high cost and medical risk of a corrective procedure rotative misorientations are largely tolerated. This constitutes suboptimal treatment of the patient.

An initial possibility for aligning the bone fracture fragments is to give an assistant physician the task of exerting the forces and repositioning the fragments by pulling and aligning the distal end of the extremity. During this, the operator aligns and fastens the osteosynthesis material with the aid of an X-ray image intensifier. In doing so the assistant physician and operator stand very close to a source of X-rays for a longer period and are therefore exposed to increased radiation. Particularly because of constant small movements by the assistant physician, the X-ray image has to be constantly updated.

The problem with this is that alignment of the bones can take place with a rotative error which is not detected during the operation. A malposition of up to 15° is therefore considered as acceptable. For the patients, this means for example internal/external rotation of the foot of up to 15° which postoperatively can lead to complications or increased wearing of the joint.

In the case of a fracture of the lower extremities, a patient can also be placed on an extension table and the distraction forces exerted on the patient via this. The advantage of this is that the repositioning of the extremity is considerably more stable.

A drawback is that pre-operative positioning is laborious and partially contra-indicated in the case of patients with multiple traumas. There have also been reports of nerve damage and soft tissue lesions in the perineum.

A further possibility is the use of a mechanical external fixator. This is a mechanical device with which the fragments are fixed to each other by means of Schanz screws/Kirschner wires via extracorporeal rods. Systems are known in which the rods can be adjusted by means of spindles.

A further possibility is the use of robotic assistance systems. Here, usually the distal fragment of the fracture fragments is positioned via suitable fastening or gripper systems in relation to the proximal fragment by way of a robotic system. The disadvantage of this is that this is a very time-consuming procedure as the robots, as well as an appropriate optical navigation system, have to be initialised and registered. In most cases, these processes require 3D X-ray images which can only be produced by CT. Such techniques can therefore only be used if 3D X-ray images with reference bodies are available, specialists who can proficiently operate the systems are present on site, and the patient's condition is not at risk/is stable.

It is the aim of the invention to provide a device for the repositioning of bone fracture fragments with the help of which accurate repositioning of the bone fracture fragments is made possible in a simple manner.

This is achieved through a device with the features of claim 1. Advantageous further embodiments of the invention are set out in the dependent sub-claims.

In accordance with the invention, the device has an electric actuator unit for adjusting the second arm. The device also has an operating element for controlling the adjustment of the second arm via the electric actuator unit.

This means that the repositioning of the bone fracture fragments relative to each other can be automatically carried out by the device in a simple manner. For this, the first bone fracture fragment is fastened to the first arm and the second bone fracture fragment to the second arm. By means of the actuator unit, with the aid of the operating unit, the second bone fracture fragment is then moved into the required position in a motorised manner.

This has the advantage that the forces required for repositioning do not have to be exerted manually, through which a considerably more precise repositioning can take place, thereby preventing malpositions. In addition, no persons need to be present in the X-ray area during the repositioning.

Compared with robotic systems the device has the advantage that it is of much simpler and compact construction.

In a particularly preferred embodiment, on the end of the first arm facing away from the carrier unit, a first holder unit for holding Schanz screws and/or, on the end of the second arm facing away from the carrier unit, a second holder unit for holding Schanz screws is/are provided. In this way the Schanz screws previously inserted into the bone fracture fragments can be fastened to the arms in a simple manner, so that via alignment of the arms relative to each other the bone fracture fragments held by the Schanz screws can then be brought into the desired position.

It is particularly advantageous if, with the help of the actuator unit, the second arm is adjustable in such a way that the position of the second actuator unit relative to the carrier unit can be adjustable at will, i.e. in all directions if possible.

More particularly, with the help of the actuator unit the second arm can be adjusted in position and alignment in such a way that the second holder unit is adjustable about all six degrees of freedom of three-dimensional space. The second holder unit can thus be moved along three axes orthogonally positioned with regard to each other and also be turned about all three axes. This achieves that any desired alignment of the bone fracture fragments is possible so that every necessary position can be set.

The carrier unit also comprises a fastening unit for fastening the device to an operating table. This has the advantage that the device can be attached to the appropriate operating table whenever it is needed. More particularly, the device is designed in such a way that a friction connection is produced so that when the patient's position is changed the forces remain approximately constant.

The fastening unit is also designed in such a way that it has an articulated joint through which the device can be turned about at least one axis relative to the operating table, so that, more particularly, the legs can be splayed accordingly.

The fastening unit is also designed in such a way that the device can be attached to standard interfaces for holding leg plates.

In a particularly preferred embodiment, the first arm has at least one manually adjustable articulated joint. Preferably, several manually adjustable articulated joints are provided. This achieves that the position and alignment of the first holder unit can also be changed, so that the necessary positioning of the first bone fracture fragment can take place. More particularly, manually operated securing units are provided on the articulated joints to secure the articulated joints, so that after manual alignment of the first arm this can be fixed in the desired position.

The first arm is thus purely manually adjustable. In an alternative embodiment of the invention, a further electric actuator unit, more particularly one motor or more motors, can be provided for adjusting the first arm.

The articulated joint(s) of the first arm is/are designed so that the first holder unit can be adjusted about six degrees of freedom.

The first arm can, more particularly, be an arm as is described in patent DE 102 09 209 B4 or in patent application DE 10 2012 112 716 A1. The design and function of the arms are set out in this description with reference to patent DE 102 09 209 B4 and patent application DE 10 2012 112 716 A1.

It is also advantageous if the carrier unit has a rail and if the second arm has a slide which is arranged on the rail to move in the direction of the longitudinal axis of the rail. Movement of the slide, and thus of the second arm on the rail takes place in particular with the aid of the electric actuator unit.

For this, the electric actuator has a first motor, which engages with a spindle via which the slide can be moved along the rail.

In a particularly preferred embodiment of the invention, the second arm also has several articulated joints, wherein each articulated joint is assigned a motor for adjusting the articulated joint. More particularly, these motors of the electric actuator unit are each arranged directly on the articulated joint so that no elaborate force transmission over longer distances, which would lead to susceptibility to error and contamination, is necessary.

In a particularly preferred embodiment of the invention the second arm has a first, a second as well as a third articulated joint. The second arm also has at least two connection plates. The first articulated joint connects the first connection plate to the slide in an articulated manner. The first connection plate is arranged between the first and the second articulated joint, wherein the second articulated joint connects the first and the second connection plate to each other in an articulated manner. Arranged on the end of the second connection plate facing away from the second articulated joint is the third articulated joint, to which, in turn, the second holding unit is fastened.

Hereby, in a particularly simple and compact fashion, a robust arm is created which is adjustable in a motorised manner and enables movement of the second holder unit and thus of the second bone fracture fragment in all six degrees of freedom of three-dimensional space.

The electric actuator unit controlling the operating unit for adjusting the second arm is, more particularly, arranged in the region of the third articulated joint and is thus located on the end of the second arm facing away from the carrier unit.

The operating unit is designed in such a way that, when operated in a predetermined direction, the second arm is moved/turned in this direction accordingly. Through being fastened on the end of the second arm, intuitive control is achieved, i.e. the operator only has to move the operating element in the way that the second arm is to be moved. In this way, precise adjustment of the second arm is possible without the need for a great deal of training and experience.

It is also advantageous if the first articulated joint is a unidirectional joint, i.e. that the first articulated joint only permits rotation about a first rotational axis. The second articulated joint is also unidirectional and thus allows rotation about a second rotational axis. It is particularly advantageous if the first and second rotational axes run in parallel to each other.

More particularly, the third articulated joint is designed in such a way that it allows rotation about three axes of rotation arranged orthogonally with regard to each other. In particular, one of these three axes of rotation is aligned in parallel to the first and the second axis of rotation. Thus, via these three simply and stably constructed articulated joints adjustment in five degrees of freedom is made possible. The sixth degree of freedom is made possible by the movement of the slide, and thus of the second arm on the rail.

It is also advantageous if the operating element comprises a joystick which allows simple and intuitive operation. More particularly, the operating element itself has six degrees of freedom so that, on operating the operating element about one of these degrees of freedom of the arm, the bone fracture fragment also moves about this degree of freedom in a corresponding manner. Envisaged as the operating element is a so-called 6 DoF sensor which makes operating input for controlling six degrees of freedom simple.

Additionally, it is advantageous if an upper leg cushion for supporting an upper leg and/or a lower leg cushion for supporting a lower leg is/are attached to the carrier unit. More particularly, the fastenings of the cushions are designed so that the cushions can be removed. Thus, after every step of the operation, for example, the insertion of an intramedullary nail, the necessary supporting of the broken leg can take place or the necessary access to the leg assured.

A further aspect of the invention relates to an operating table, wherein at least one device of the aforementioned type is detachably fastened to this operating table. It is of particular advantage if the device is fastened to the operating table via a friction-type fastening.

Further features and advantages of the invention are set out in the following description which explains the invention in more detail by way of examples of embodiment in conjunction with the attached figures.

In these:
- Figure 1: shows a schematic, perspective view of a device for repositioning bone fracture fragments;
- Figure 2: shows a further schematic, perspective view of the device in accordance with figure 1;
- Figure 3: shows a further schematic, perspective view of the device in accordance with figures 1 and 2;
- Figure 4: shows a schematic view of an arrangement with a section of an operating table and the device in accordance with figures 1 to 3;
- Figure 5: shows a view from above of the arrangement in figure 4;
- Figure 6: shows a view from above of the arrangement in figures 4 and 5 with changed orientation of the device for repositioning the bone fracture fragments;
- Figure 7: shows a schematic, perspective view of the arrangement in accordance with figure 5;
- Figure 8: shows a further schematic, perspective view of the arrangement in accordance with figures 4 to 6 with the upper leg cushion in place;
- Figure 9: shows a schematic, perspective view of the arrangement in accordance with figures 4 to 8 with the upper leg cushion and lower leg cushion in place;
- Figure 10: shows a perspective detailed view of the device in accordance with figures 1 to 3;
- Figure 11: shows a further perspective detailed view of the device in accordance with figures 1 and 3;
- Figure 12: shows a further detailed view of the device in accordance with figures 1 to 3;
- Figure 13: shows a sequence plan for controlling the actuators for repositioning bone fracture fragments.

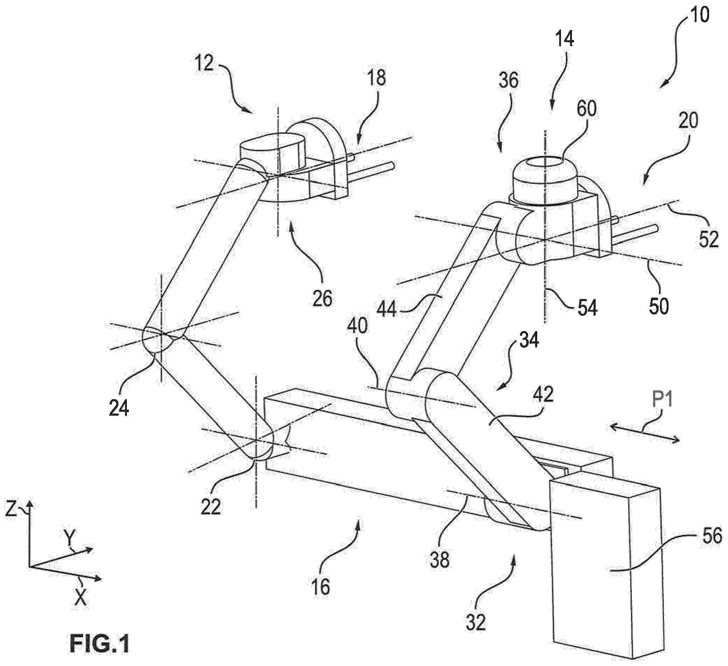

Figures 1 to 3 each show a schematic, perspective view of a device 10 for repositioning bone fracture fragments.

In fractures of human bones, more particularly of large hollow bones, the fragments have to be aligned/repositioned with regard to each other. Only after this has been done can the bone fragments be fixed by means of plates and/or intramedullary nails.

For repositioning the bone fracture fragments, the device 10 comprises a first arm 12 as well as a second arm 14, a first end of each of which is attached to a carrier unit 16. On the ends opposite the carrier unit 16, a respective holder unit 18, 20 is arranged, to which Schanz screws inserted into the bone fracture fragments can be fastened.

Initially, the Schanz screws of a first bone fracture fragment are fastened to the first arm 12, more particularly to the first holder unit 18. The first holder unit 18, and thereby the first bone fracture fragment attached to it, is then brought into the desired position. For this the first arm 12 has three articulated joints 22, 24, 26 which are manually adjustable. More particularly, the articulated joints 22, 24, 26 are designed so that the first holder unit 18, and hence the bone fracture fragment attached to it, can be moved all three spatial directions as well as turned in three directions. In this way the alignment of the first bone fracture fragment can be changed as desired.

The second bone fracture fragment is then attached via Schanz screws to the second holder unit 20 and aligned with the help of the second arm 14.

The second arm 14 also has three articulated joints 32, 34, 36, via which the position and alignment of the second holder element 20 can be varied.

The first articulated joint 32 is a unidirectional joint that enables rotation about a first axis of rotation 38. The second articulated joint 34 is also unidirectional and allows rotation about a second rotational axis 40. A first connection plate 42 is arranged between the first and the second articulated joint.

In addition, a second connection plate 44 is arranged on the second articulated joint 40, via which the second articulated joint 34 is articulately connected to the first connection plate 42. Arranged on the end of the second connection plate 44 facing away from the second articulated joint is the third articulated joint 36.

The third articulated joint 36 is designed as an articulated joint assembly with three separately-driven individual articulated joints, so that the articulated joint 36 allows rotation about three axes of rotation 50, 52, 54 arranged orthogonally with regard to each other.

More particularly, the axes of rotation 38, 40 and 50 are arranged in parallel with each other.

Through the above-described structure it is achieved that through the three articulated joints 32 to 36 alone adjustment about five degrees of freedom is possible.

In order to also allow adjustment about the remaining, sixth degree of freedom, the end of the second arm 14 facing away from the second holder unit 20 is arranged on a slide which can be moved on a rail of the carrier unit 16 in the direction of the double arrow P1.

Both the movement of the second arm 14 on the rail of the carrier unit 16 and the adjustment of the articulated joints 32 to 36 takes place via electric actuators, not shown, which are controlled by a control unit 56.

In other examples of embodiment the first articulated joint 32 and/or the second articulated joint 34 can also be in the form of an articulated joint assembly so that movement about more than one axis of rotation is made possible for these joints.

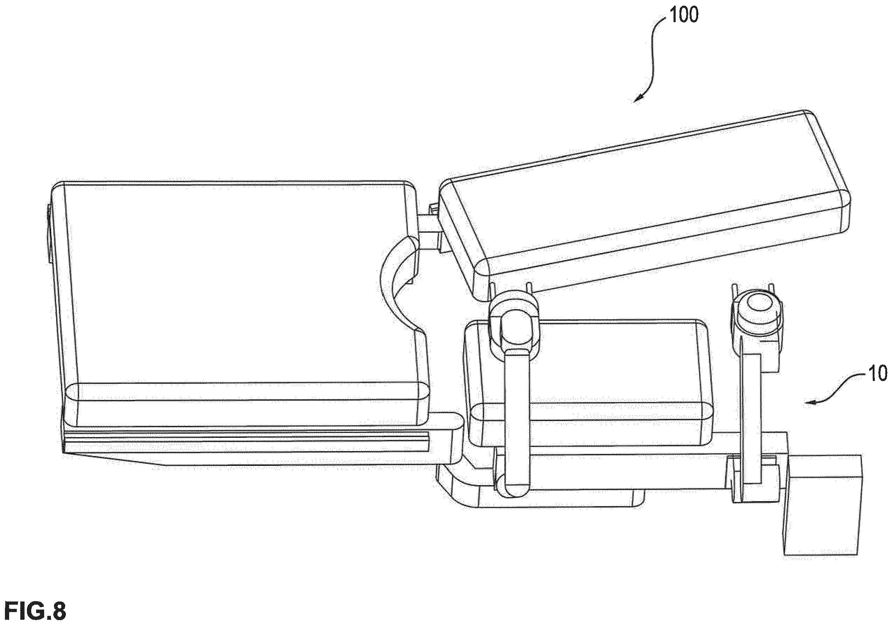

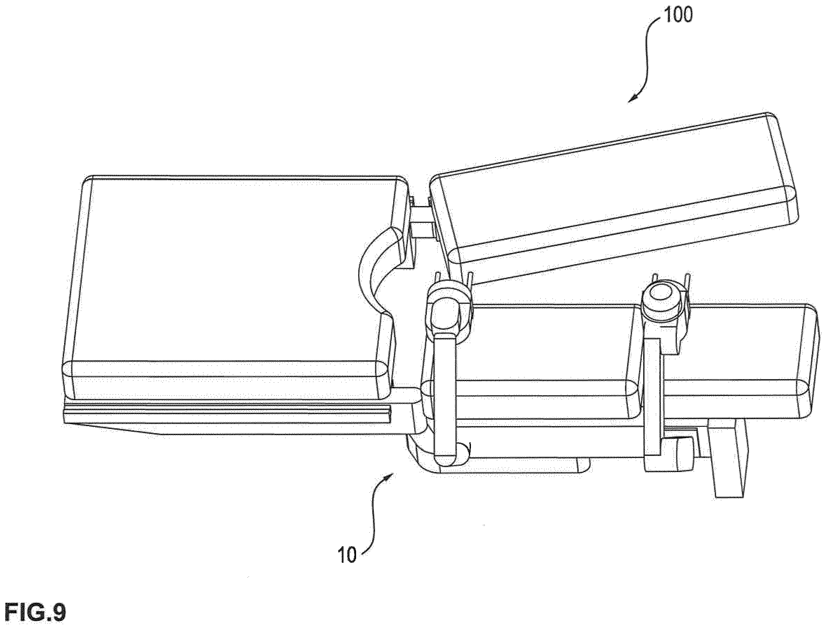

In addition to the mechanical assembly described and shown in the figures, the device 10 comprises a control and operating unit with an operating element 60, the control unit 56 and the electric actuators, which are not shown separately in figures 1 to 9. The electric actuators each have suitable motor transmission combinations. More particularly, a first electric actuator for moving the second arm 14 on the rail of the carrier unit 16 is provided. In addition, five further electric actuators are present, wherein each of these actuators is for adjusting one of the articulated joints 32 to 36 and arranged directly on it.

Furthermore, arranged on the end of the second arm 14 on which the holder unit 20 is also arranged, is an operating element 60 via which the electric actuator unit 56 is controlled, so that via this operating element 60 the adjustment of the second arm 14 can be controlled by the operator.

The operating element 60 is, in particular, a so-called 6 DoF (degrees of freedom) sensor, which itself can be operated in six degrees of freedom, wherein on operation of the sensor about one of these degrees of freedom the second arm is moved accordingly, so that intuitive control is possible.

The cap of the operating element 60 can, more particularly, be moved in the direction of the three axes 50, 52, 53, which results in a corresponding movement of the second holder unit 20 and thus of the second bone fracture fragment. In addition, the cap can be turned and tilted in two directions, which is converted into a corresponding rotary movement.

Depending on which treatment of the fracture is being carried out, different positioning of the broken extremity before, during and after the operation is necessary. In order to assure this, various cushions can be applied to and also removed from the carrier unit 16. This is advantageous in order to allow intraoperative access to the fracture and to remove objects that negatively affect the X-ray image to be removed from the direct radiation beam.

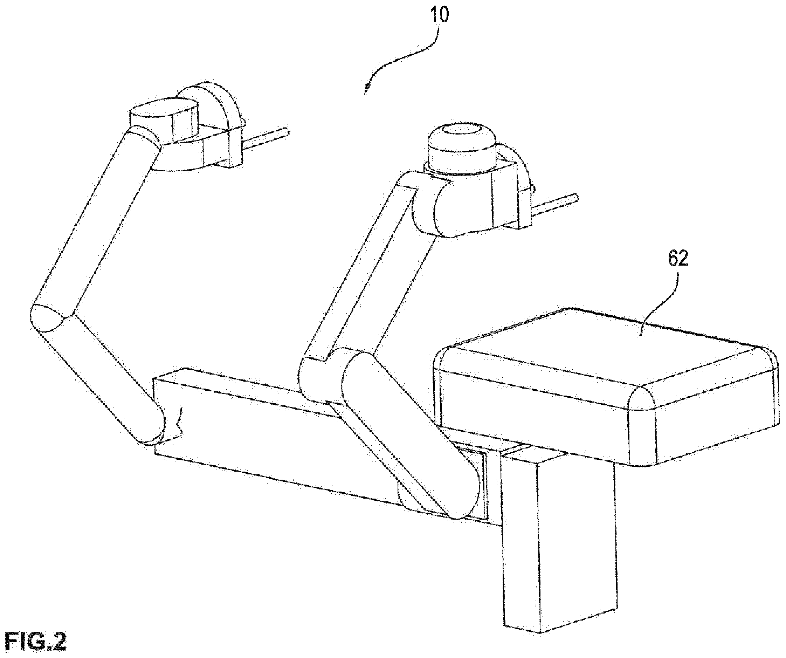

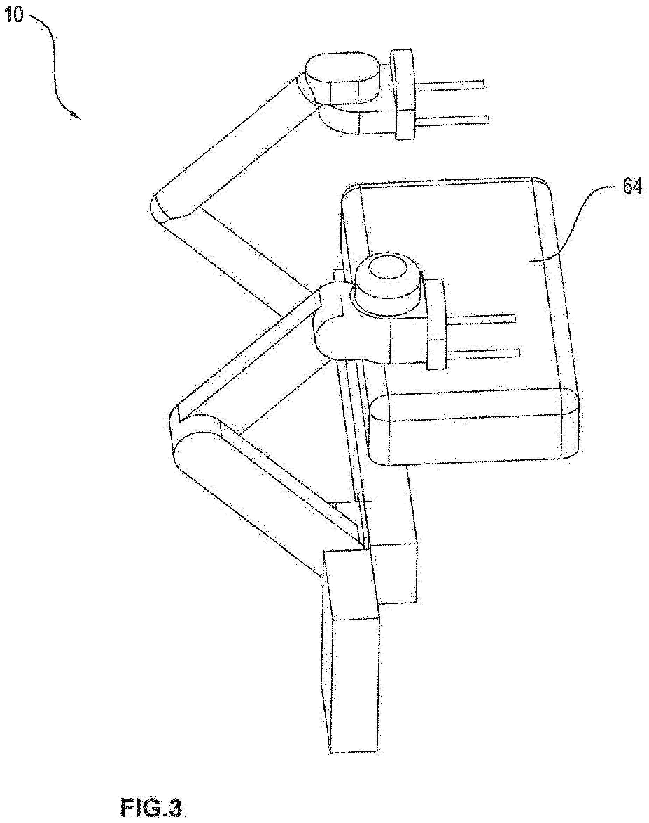

In the form shown in figure 1, no cushion at all is fastened to the carrier unit 16. In the embodiment shown in figure 2, on the other hand, a lower leg cushion 62 is provided. The embodiment in figure 3 shows an upper leg cushion 64 firmly fastened to the carrier unit 16. Alternatively, both cushions 62, 64 can of course be provided.

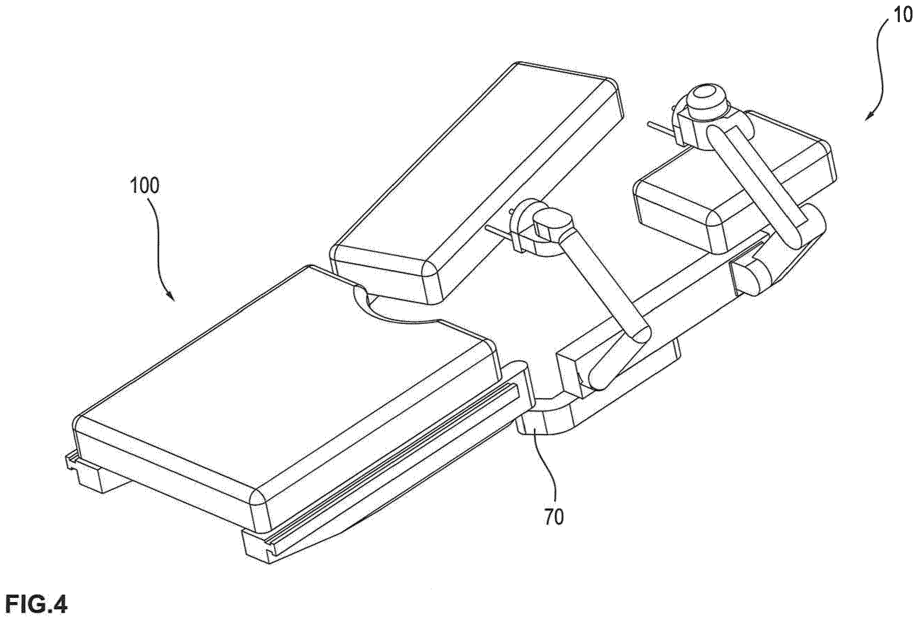

As shown in figures 4 to 9, the device 10 can be fastened to an operating table 100. For this, the device 10 has a fastening unit 70, which, in particular, allows articulated fastening to the operating table 100 by a friction-type connection.

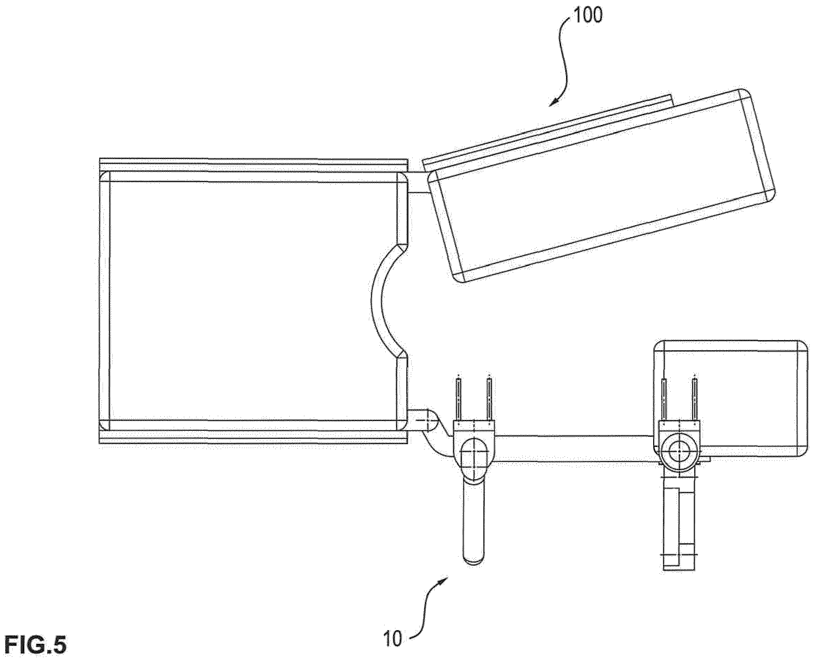

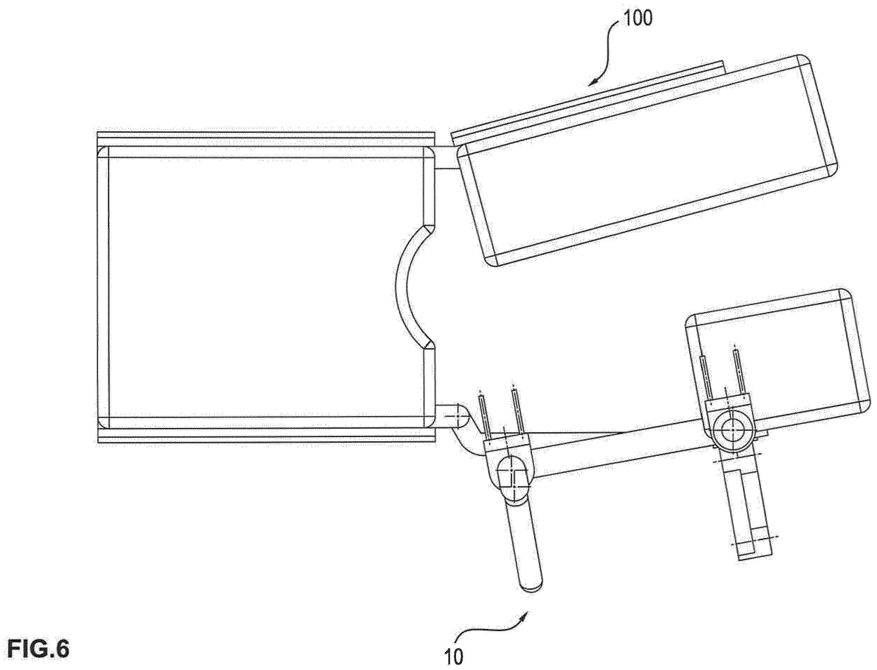

As can be seen in figures 5 and 6, the connection between the operating table 100 and the device 10 is such that swivelling of the device 10 is possible, so that the leg placed on it can be positioned accordingly.

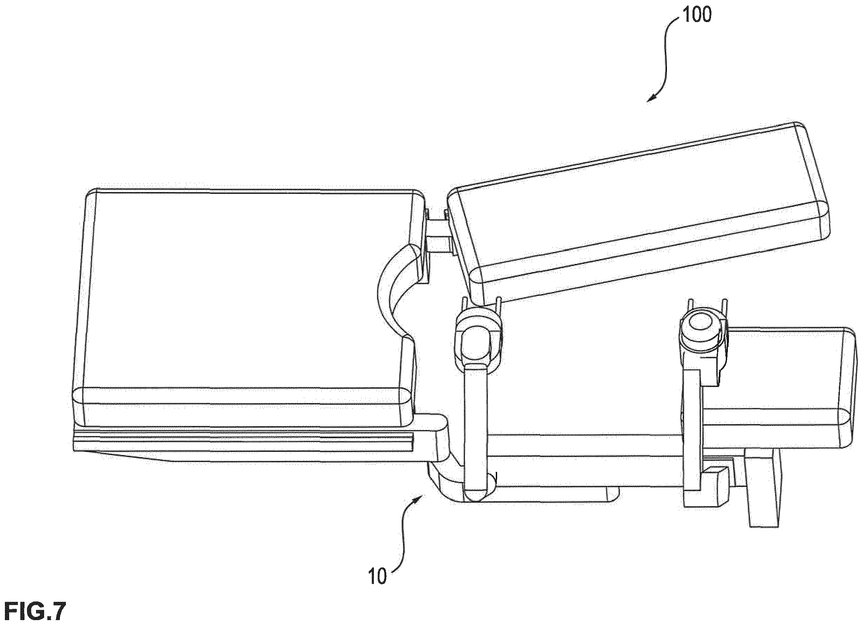

In figures 7 to 9, further embodiments are shown, wherein a different combination of the applied cushions 62, 64 is shown in each embodiment.

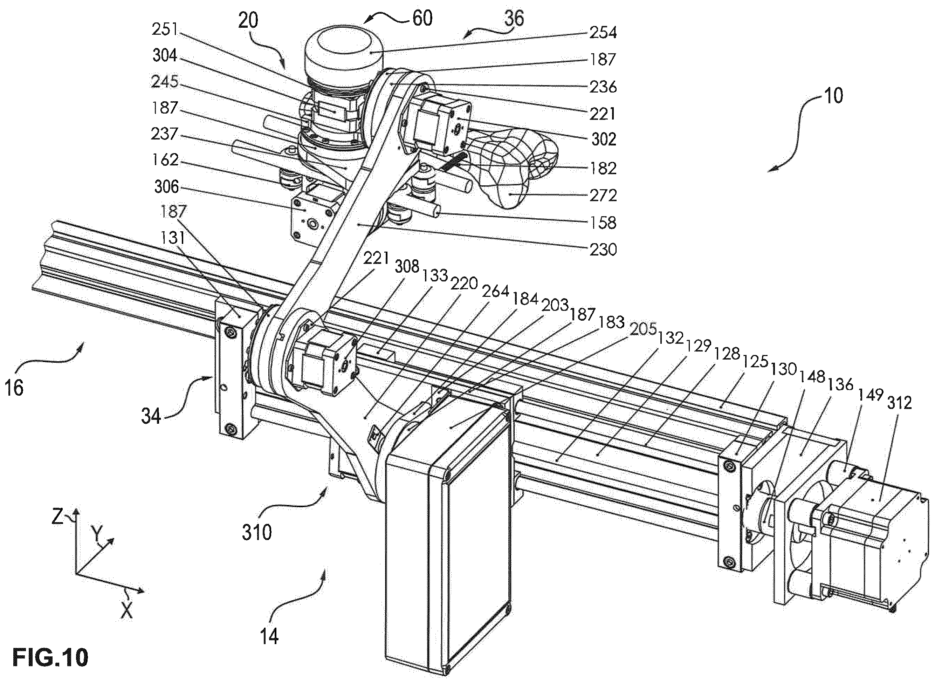

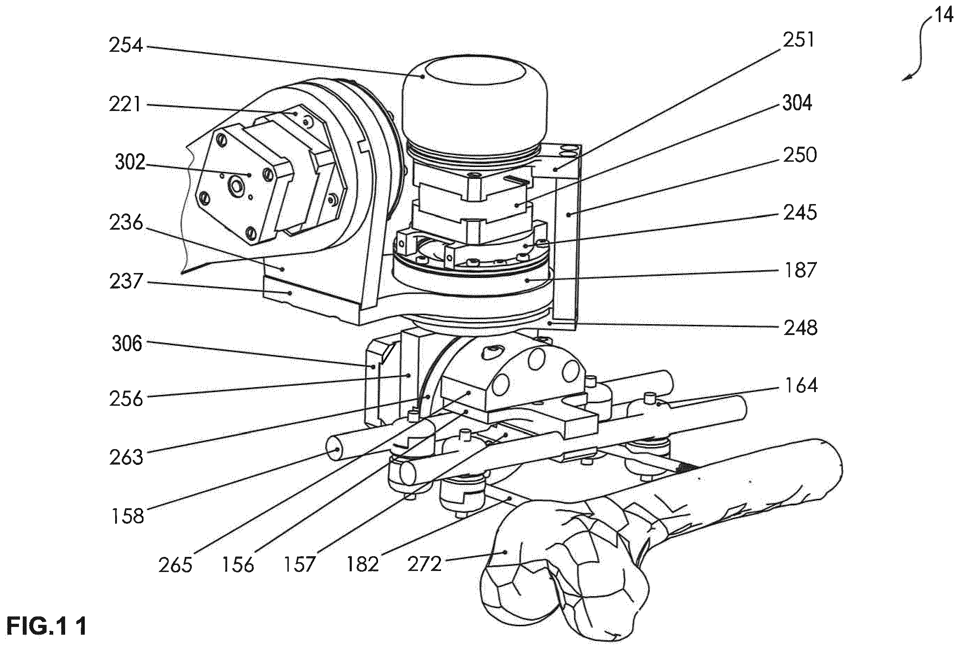

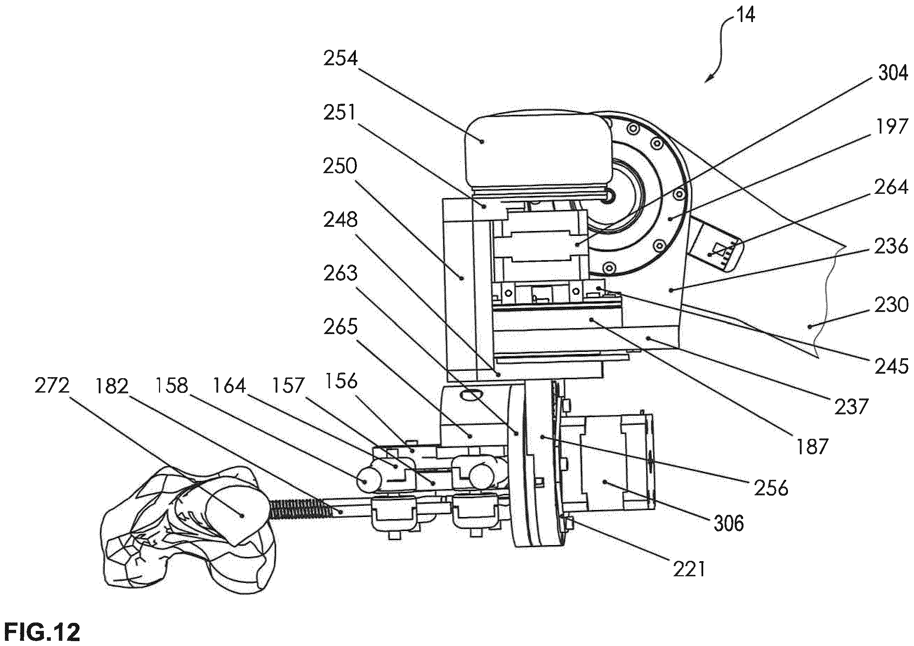

Figures 10 to 12 each show detailed views of the device 10, wherein in figure 10 the second arm 14, a part of the carrier unit 16 and the rail of the carrier unit 16 are shown. Figures 11 and 12 each show the third articulated joint 36 as well as the second holding unit 20.

A fundamental component of the device is a linear track. It is aligned with its main axis along the X-axis of the coordinate system of the device 10. The Y-axis, which is perpendicular to the X-axis, points in the depth direction. The Z-axis, perpendicular to the other two axes, points upwards.

The track comprises a support structure 129 with flanged-on terminals 130, 131 at both ends. Running between these terminals are two guide rails 128 on which a slide 183 moves. The slide 183 is driven by the threaded rod 132. The rotation of the threaded rod 132 is converted by a threaded sleeve incorporated in the slide 183 into a self-inhibiting translation. The threaded rod 132 ends on one side of the support structure in a bearing which is located in one of the two terminals 130, 131. The other terminal 130 is also designed in the same way, but to a certain extent gives the spindle clearance to the outside. On this part of the spindle, an end piece of an Oldham coupling 148 is clamped on. The other end of the coupling is clamped to the shaft of an electric motor 312. The motor 312 is fastened in mechanical dampers 149. These, in turn, are attached to a holder structure 136 consisting of three plates perpendicular to each other. The holder structure is screwed to the outer side of the terminal 130 which assures access to the spindle. In this way, through rotation of the motor axle, the slide 183 can be moved linearly along the spindle axle.

Attached to the slide 183 is a multiple-joint arm structure which allows the holder unit 20 attached to the end effector to move in 5 degrees of freedom. Preferably, the first three axes of the articulated arm are aligned in parallel. With these three axes, three of the degrees of freedom can already be set.

A bottom place is attached to the slide and perpendicularly thereto a base plate 184. It comprises a gear module 187 which is driven by an electric motor 310 and has an integrated cross-roller bearing for diverting the forces and torques and constitutes the articulated joint 32. The plate structure 220 is mounted on the end face of the gear output. Accommodated in a recess, this plate has the circuit board 264 of the angle measuring system belonging to the articulated joint 32. At the upper end of the plate 220, there is an electric motor 308 of the second articulated joint 34 on the same side as the gear output of the basic articulated joint 32. This is attached by means of a flanged plate. Attached on the opposite side of the plate 220 is the gear mechanism of the articulated joint 34. This is also in contact with the end surface of the gear output on the plate 220. The plate 230 is applied to the end surface of the drive of the gear mechanism. The board of the angle measuring system of articulated joint 34 is also located in a corresponding recess on the other side of the plate 230, relative to the recess of the first-mentioned angle measuring system.

On the upper side of the plate 230, an electric motor 302 is attached in the same alignment as the electric motor 308 of the articulated joint 34 by means of a flanged plate. In the same position on the plate 230 on the opposite side, the output of the gear mechanism 187 of the articulated joints 36 is fixed with its end surface on the plate 230. On the end surface of the drive wheel 187, a mounting plate 236 is applied.

Attached to this at a right angle on the side of the gear mechanism 187 is a further plate 237. This provides a mounting for the gear mechanism 187 used downwards on the Z-axis of the device 10. Attached above this gear mechanism 187 by means of an adapter flange 245 is a further electric motor 304 for actuating the articulated joint 36.

Furthermore, above the motor 304, a 6 DoF sensor with an operating element is attached on a platform 251. The alignment of its axes corresponds to the arrangement of the X-axis, Y-axis and Z-axis of the device 10 in the normal state.

Under the gear mechanism 187 of the Z-axis, a mounting plate 256 is perpendicularly attached on the end surface of the gear output. On its rear, the plate 256 accommodates an electric motor 306 by means of an flanged plate. The shaft of this is non-rotatably connected to a gear mechanism, the direve wheel of which is fastened to the end surface of mounting plate 256. This combination causes about an adjustment of the articulated joint 36 about the Y-axis.

On the output wheel of the latter gear mechanism is a mounting plate 263 which constitutes the mechanical connection for the pin holder. The pin holder initially consists of a conventional external fixator configuration. This consists of two rods 158 to which two Schanz screws 182 are clamped by means of special clamping jaws 164. The two basic holders should be applied in as parallel a manner as possible. Ideally, the Schanz screws are fixed in the clamping jaws perpendicularly to the two basic rods in a plane parallel to the plane through the two basic carriers.

Both Schanz screws are screwed into the distal femur segment 272. Here, it must be taken into account that in the case of intramedullary nailing, the Schanz screws close to the fracture gap can only be inserted monocortically. For insertion of the nail the medullary space must be free of obstacles.

To hold the basic rod system the device 10 has a clamping jaw holder, comprising a mountable cap 157 and a base plate 156 into which the cap 157 is screwed.

By means of a transition piece 265 the base plate 156 is attached on its underside at right angles to the mounting plate 263. For this, the transition piece 265 has three drilled holes for assembly on said mounting plate 263 and two drilled holes perpendicular thereto for assembly of the base plate 156 of the pin holder.

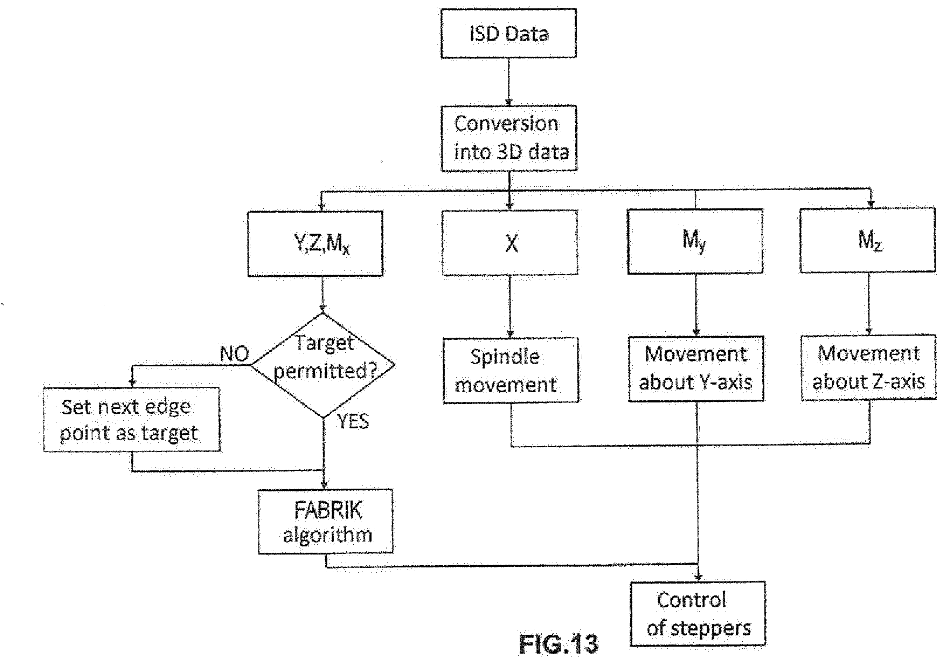

Figure 13 shows a sequence plan for controlling the actuators for repositioning bone fracture fragments. In connection with operating input by way of the operating element 60, ISD data are generated which are then converted into 3D data. When the device 10 is in operation, through applying power to the motors and through braking, a holding torque is exerted in each articulated joint 32, 34, 36. This results in permanent holding of the position and orientation of the pin holder and the Schanz screws connected thereto. Ideally, this also results in the permanent retention of the distal femur fragment. If the user changes the position or orientation of the cap of the 6DoF sensor, this is converted into a movement. This is characterised in that an attempt is made to execute the user requirement as intuitively as possible.

If the user requires a translation of the pin holder in the positive X-direction, he pushes the operating element 60 with the cap 254 in this direction with his hand on top of it. In this case, the user requirement is directly implemented in a controlling of the motor on the spindle. If the user requires a translation in the Z or Y-direction he also pushes the cap in one of these directions. This requirement is implemented by means of the articulated joint arm structure. At this time, an inverse problem has to be solved. The translation is known, it is recorded in the 6DoF sensor, but the angle settings required therefore have to be individually calculated. For this, an iterative process named FABRIK is used. The translation desired by the user is delivered unit-less by the sensor and converted into a speed. For the known duration of controlling the motor, the travelled path is calculated and added to the position of the target point.

For controlling the motors 302 to 312, a speed trajectory is generated. This consists of a linear acceleration phase with a defined gradient until the maximum speed is reached. It is held until a linear deceleration ramp with a defined gradient sets in and the speed has fallen to zero.

If the user continues to maintain his input during the movement of the system, the start of the deceleration ramp is recalculated and it is started correspondingly later if it has not yet been reached. If the system is already in the deceleration phase, an acceleration ramp necessary for reaching the maximum speed with a defined gradient is calculated and implemented. If the user ends his entry, the previously calculated movement trajectory is runs until completion. Thereafter the achieved positioning is held permanently. The same procedure applies to changing the alignment of the end effector. Here in, the user turns the cap 254 about at least one of the axes. If this is a rotation about the Y-axis or Z-axis - designated My and Mz - the relevant drive unit 302 to 306 of the articulated joint 36 is directly actuated.

However, if this is a rotation about the X-axis - designated Mx -, more particularly by means of the FABRIK algorithm a new configuration of the angle settings of the articulated arm is calculated. The participating articulated joints are then actuated as described. On completion of the process, the alignment and position of the end effector is in turn held without user input.

The envisaged intraoperative use of the device 10 takes place as follows. First of all, the operator has to fit each fragment with two Schanz screws. These should preferably be inserted in one plane, but do not have to be parallel to each other. In addition, one screw should be inserted quite close to the fracture gap and one screw quite far away from the fracture gap. In accordance with the procedure of applying an external fixator, the screws are clamped onto two parallel rods with special clamping jaws. By means of a clamp mounting, these rods are then clamped onto the holder on the end effector of the relevant articulated arm.

On completion of these measures, the device 10 can be positioned and fixed on the operating table. Alternatively the device 10 can be fixed to the operating table before the start of positioning and only at that point brought into operation. This means that the passive, first articulated arm is initially brought into position and manually fixed. The active, second articulated arm is then moved into the desired position through input via the sensor element. Via the screw clamping, the rods of the external fixator configuration are connected with the end effector of the relevant articulated arm. For this, the rods are moved into the basic shell of the clamping device, the clamping cap positioned above them and both are pulled towards each other through turning of the centrally arranged screw.

If a play-free mechanical clamping connection is created, the operator can loosen individual joints of the passive arm and newly align the proximal fragment. After satisfactory alignment, the arm is completely fixed. The thus produced retention of the position and orientation of the proximal fragment serves as a reference for the alignment and positioning of the distal fragment. For this, the operator uses the motorised articulated arm and changes the alignment and position of the end effector through acting on the sensor cap by means of the hand placed on it. After satisfactory alignment, the operator releases the sensor. The device now keeps the set position and orientation constant. By way of X-ray imaging, the operator can assure the quality of his repositioning. Therein, for protection against radiation exposure, the entire operating team can assume a certain distance from the radiation beam. After evaluation of the produced images, corrections can be carried out through renewed positioning. On completion of the positioning, the guide wire can be introduced and, if applicable, the medullary space drilled open. If nail application is being carried out without drilling open and without guide wire, the nail can inserted at this point. After the nail has passed the fracture gap the device must be removed. This is done in reverse order to the application of the device. First of all, the clamping connection with the device with the external fixator must be loosened, and the Schanz screws are then removed. Finally, the procedure is conventionally ended in that the nail is fully inserted in the usual manner, locked, and the closing cap applied - if required - and all wounds are closed.

When plating the fragments, the device remains in place during this stage of treatment.

Through the above-described device, an increase in the precision of positioning the bone is achieved in particular.

In addition, this avoids the need for parts of the operator's body to remain in the radiation beam of an X-ray device during the process of producing the X-ray images.

Furthermore, the operator is able to intuitively control the described system.

In comparison with other robotic systems which are controlled by joysticks firmly fixed in space, the special feature in this case is that the sensor is located directly on the end effector. This results in the following scenario: The surgeon or the assistant physician covers with one hand the area of the extremity which is distal of the fracture and with the other hand the sensor element which is arranged very close to the extremity and undergoes no movement relative to the extremity. In this way, the physician can move the area of the extremity in the same way as he would do without using the device. This procedure is very intuitive and does not require long learning times. A further advantage is that the physician has a hand on the extremity at all times and therefore receives permanent haptic feedback in the same way as he would in purely manual repositioning, with the advantage that no high tensile forces have to be applied to the extremity. Through the de-coupling of the high tensile force produced by the patient's muscles, very delicate and precise work can be carried out, which is associated with a better outcome.

Moreover, the device 10 is portable, small and light in weight, can be mounted on the universal rail of an operating table or the leg plate interface of an operating table and enables integration into leg plates.

In comparison with robotic systems, the device 10 requires no navigation and initialisation, no current CT data for the patient and no specially trained personnel. Over and beyond this, the method is considerably less costly and time-consuming and allows intuitive control of the system.

## Claims

1. Device for repositioning bone fracture fragments comprising
a carrier unit (16),
a first arm (12) attached to the carrier unit (16) for holding a first bone fracture fragment, and
a second arm (14) attached to the carrier unit (16) for holding a second bone fracture fragment and comprising a first, a second and a third articulated joint (32 to 36), wherein the device (10) has an electric actuator unit (56) for adjusting the second arm (14), and wherein the device (10) has a manually operatable operating unit (60) for controlling the adjustment of the second arm (14) via the actuator unit (56), the operating unit (60) being arranged in the region of the third articulated joint (36).

2. Device (10) according to claim 1, **characterised in that** on the end of the first arm (12) facing away from the carrier unit (16) a first holder unit (18) for holding Schanz screws and/or on the end of the second arm (14) facing away from the carrier unit (16) a second holder unit (20) for holding Schanz screws is/are provided.

3. Device (10) according to claim 2, **characterised in that** with the help of the actuator unit (56) the second arm (14) is adjustable in such a way that the position of the second holder unit (20) relative to the carrier unit (16) is adjustable in several directions,
wherein, preferably, the second arm (14) is adjustable with the help of the actuator unit (56) in such a way that the position and alignment of the second holder unit (20) is adjustable about all six degrees of freedom of three-dimensional space.

4. Device (10) according to any one of the preceding claims, **characterised in that** the carrier unit (16) has a fastening unit (70) for fastening the device (10) to an operating table (100),and/or in that the end of the first arm (12) facing the carrier unit (16) is firmly fastened to the carrier unit (16).

5. Device (10) according to any one of the preceding claims, **characterised in that** the first arm (12) has at least one manually adjustable articulated joint (22 to 26), preferably several manually adjustable articulated joints (22 to 26).

6. Device (10) according to claim 5, **characterised in that** a securing unit for securing the articulated joint (s) (22 to 26) in a set position is provided, and/or
**in that** the articulated joint (s) (22 to 26) is/are designed in such a way that the first holder unit (18) can be adjusted about six degrees of freedom with the help of the first arm (12).

7. Device (10) according to any one of the preceding claims, **characterised in that** the carrier unit (16) comprises a rail and that the second arm (12) has a slide, which is borne on the rail and is moveable in the longitudinal direction of the rail with the aid of the actuator unit (56),
wherein, preferably, the actuator unit (56) comprises a first motor for moving the slide.

8. Device (10) according to any one of the preceding claims, **characterised in that** assigned to each articulated joint (32 to 36) of the second arm (14) is a motor for moving the respective articulated joint (32 to 36).

9. Device (10) according to claim 7, **characterised in that** the second arm (14) has two connection plates (42, 44), wherein the first articulated joint (32) is arranged on the slide, the first connection plate (42) between the first and the second articulated joints (32, 34) and the second connection plate (44) between the second and the third articulated joints (34, 36).

10. Device (10) according to claim 2, **characterised in that** the second holder unit (20) is arranged on the third articulated joint (36).

11. Device (10) according to any one of the preceding claims, **characterised in that** the first articulated joint (32) has a first axis of rotation (38) and the second articulated joint (34) has a second axis of rotation (40) and that the first and second axes of rotation (38, 40) run parallel to each other, or in that the third articulated joint (36) has three axes of rotation (50 to 54) arranged orthogonally with regard to each other.

12. Device (10) according to any one of the preceding claims, **characterised in that** the operating unit (60) comprises a joystick.

13. Device (10) according to any one of the preceding claims, **characterised in that** the operating unit (60) has six degrees of freedom, and/or in that on the carrier unit (16) an upper leg cushion (64) and/or a lower leg cushion (62) is/are detachably fastened.

14. Operating table (100), **characterised in that** on the operating table (100) at least one device (10) according to any one of claims 1 to 13 is detachably fastened.

15. Operating table (100) according to to claim 14, **characterised in that** the device (10) is attached via a friction-type fastening.

## Patentansprüche

1. Vorrichtung zum Repositionieren von Knochenbruchfragmenten, umfassend
eine Trägereinheit (16),
einen ersten, an der Trägereinheit (16) angebrachten Arm (12) zum Halten eines ersten Knochenfrakturfragments, und
einen zweiten, an der Trägereinheit (16) angebrachten Arm (14) zum Halten eines zweiten Knochenbruchfragments und umfassend eine erste, eine zweite und eine dritte Gelenkverbindung (32 bis 36),
wobei die Vorrichtung (10) eine elektrische Betätigungseinheit (56) zum Verstellen des zweiten Arms (14) aufweist, und
wobei
die Vorrichtung (10) eine manuell bedienbare Bedieneinheit (60) zum Steuern der Verstellung des zweiten Arms (14) über die Betätigungseinheit (56) aufweist, wobei die Bedieneinheit (60) im Bereich der dritten Gelenkverbindung (36) angeordnet ist.

2. Vorrichtung (10) nach Anspruch 1, **dadurch gekennzeichnet,**
**dass** am Ende des ersten Armes (12) abgewandt von der Trägereinheit (16) eine erste Aufnahmeeinheit (18) zum Aufnehmen von Schanzschen Schrauben und/oder am Ende des zweiten Arms (14) abgewandt von der Trägereinheit (16) eine zweite Aufnahmeeinheit (20) zum Aufnehmen von Schanzschen Schrauben bereitgestellt sind/ist.

3. Vorrichtung (10) nach Anspruch 2, **dadurch gekennzeichnet,**
**dass** mit Hilfe der Betätigungseinheit (56) der zweite Arm (14) derart verstellbar ist, dass die Position der zweiten Aufnahmeeinheit (20) in Bezug auf die Trägereinheit (16) in mehreren Richtungen verstellbar ist,
wobei vorzugsweise der zweite Arm (14) mit Hilfe der Betätigungseinheit (56) derart verstellbar ist, dass die Position und Ausrichtung der zweiten Aufnahmeeinheit (20) um alle sechs Freiheitsgrade des dreidimensionalen Raums verstellbar ist.

4. Vorrichtung (10) nach einem der vorhergehenden Ansprüche, **dadurch gekennzeichnet, dass** die Trägereinheit (16) eine Befestigungseinheit (70) zum Befestigen der Vorrichtung (10) an einem Operationstisch (100) aufweist und/oder dass das Ende des ersten, der Trägereinheit (16) zugewandten Arms (12) fest an der Trägereinheit (16) befestigt ist.

5. Vorrichtung (10) nach einem der vorhergehenden Ansprüche, **dadurch gekennzeichnet, dass** der erste Arm (12) mindestens eine manuell verstellbare Gelenkverbindung (22 bis 26), vorzugsweise mehrere manuell verstellbare Gelenkverbindungen (22 bis 26) aufweist.

6. Vorrichtung (10) nach Anspruch 5, **dadurch gekennzeichnet,**
**dass** eine Sicherungseinheit zum Sichern der Gelenkverbindung(en) (22 bis 26) in einer eingestellten Position bereitgestellt ist, und/oder
**dass** die Gelenkverbindung(en) (22 bis 26) derart ausgelegt ist/sind, dass die erste Aufnahmeeinheit (18) um sechs Freiheitsgrade mit Hilfe des ersten Armes (12) verstellt werden kann.

7. Vorrichtung (10) nach einem der vorhergehenden Ansprüche, **dadurch gekennzeichnet, dass** die Trägereinheit (16) eine Schiene umfasst und dass der zweite Arm (12) einen Schlitten aufweist, der in der Schiene geführt wird und in der Längsrichtung der Schiene mit Hilfe der Betätigungseinheit (56) bewegbar ist,
wobei vorzugsweise die Betätigungseinheit (56) einen ersten Motor zum Bewegen des Schlittens umfasst.

8. Vorrichtung (10) nach einem der vorhergehenden Ansprüche, **dadurch gekennzeichnet, dass** jeder Gelenkverbindung (32 bis 36) des zweiten Arms (14) ein Motor zum Bewegen der jeweiligen Gelenkverbindung (32 bis 36) zugewiesen ist.

9. Vorrichtung (10) nach Anspruch 7,
**dadurch gekennzeichnet, dass** der zweite Arm (14) zwei Verbindungsplatten (42, 44) aufweist, wobei die erste Gelenkverbindung (32) auf dem Schlitten, die erste Verbindungsplatte (42) zwischen der ersten und der zweiten Gelenkverbindung (32, 34) und die zweite Verbindungsplatte (44) zwischen der zweiten und der dritten Gelenkverbindung (34, 36) angeordnet sind.

10. Vorrichtung (10) nach Anspruch 2, **dadurch gekennzeichnet, dass** die zweite Aufnahmeeinheit (20) an der dritten Gelenkverbindung (36) angeordnet ist.

11. Vorrichtung (10) nach einem der vorhergehenden Ansprüche, **dadurch gekennzeichnet, dass** die erste Gelenkverbindung (32) eine erste Drehachse (38) aufweist und die zweite Gelenkverbindung (34) eine zweite Drehachse (40) aufweist und dass die erste und die zweite Drehachse (38, 40) parallel zueinander verlaufen, oder dass die dritte Gelenkverbindung (36) drei Drehachsen (50 bis 54) aufweist, die orthogonal zueinander angeordnet sind.

12. Vorrichtung (10) nach einem der vorhergehenden Ansprüche, **dadurch gekennzeichnet, dass** die Bedieneinheit (60) einen Joystick umfasst.

13. Vorrichtung (10) nach einem der vorhergehenden Ansprüche, **dadurch gekennzeichnet, dass** die Bedieneinheit (60) sechs Freiheitsgrade aufweist und/oder dass auf der Trägereinheit (16) ein Oberschenkelkissen (64) und/oder ein Unterschenkelkissen (62) lösbar befestigt sind/ist.

14. Operationstisch (100), **dadurch gekennzeichnet, dass** auf dem Operationstisch (100) mindestens eine Vorrichtung (10) nach einem der Ansprüche 1 bis 13 lösbar befestigt ist.

15. Operationstisch (100) nach Anspruch 14, **dadurch gekennzeichnet, dass** die Vorrichtung (10) über eine reibschlüssige Befestigung befestigt ist.

## Revendications

1. Dispositif de repositionnement de fragments de fracture osseuse comprenant
une unité de support (16),
un premier bras (12) fixé à l'unité de support (16) pour maintenir un premier fragment de fracture osseuse, et
un second bras (14) fixé à l'unité de support (16) pour maintenir un second fragment de fracture osseuse et comprenant une première, une deuxième et une troisième articulation (32 à 36),
dans lequel le dispositif (10) a une unité actionneur électrique (56) servant à régler le second bras (14), et dans lequel le dispositif (10) a une unité d'opération (60) pouvant fonctionner manuellement et servant à commander le réglage du second bras (14) au moyen de l'unité actionneur (56), l'unité d'opération (60) étant disposée dans la région de la troisième articulation (36).

2. Dispositif (10) selon la revendication 1, **caractérisé en ce qu'**à l'extrémité du premier bras (12) opposé à l'unité de support (16) une première unité de maintien (18) servant à maintenir des vis de Schanz est prévue et/ou à l'extrémité du second bras (14) opposé à l'unité de support (16) une seconde unité de maintien (20) servant à maintenir des vis de Schanz est/sont prévue(s).

3. Dispositif (10) selon la revendication 2, **caractérisé en ce qu'**à l'aide de l'unité actionneur (56) le second bras (14) est réglable de telle sorte que la position de la seconde unité de maintien (20) par rapport à l'unité de support (16) est réglable dans plusieurs directions, dans lequel, de préférence, le second bras (14) est réglable à l'aide de l'unité actionneur (56) de telle sorte que la position et l'alignement de la seconde unité de maintien (20) est réglable autour des six degrés de liberté de l'espace tridimensionnel.

4. Dispositif (10) selon l'une quelconque des revendications précédentes, **caractérisé en ce que** l'unité de support (16) est dotée d'une unité de fixation (70) servant à fixer le dispositif (10) à une table d'opération (100), et/ou
**en ce que**
l'extrémité du premier bras (12) faisant face à l'unité de support (16) est solidement fixée à l'unité de support (16) .

5. Dispositif (10) selon l'une quelconque des revendications précédentes, **caractérisé en ce que** le premier bras (12) a au moins une articulation réglable manuellement (22 à 26), de préférence de plusieurs articulations réglables manuellement (22 à 26).

6. Dispositif (10) selon la revendication 5, **caractérisé en ce qu'**une unité de fixation destinée à fixer la ou les articulations (22 à 26) dans une position définie est prévue, et/ou
**en ce que** la ou les articulation (s) (22 à 26) est/sont conçue(s) de manière à ce que la première unité de maintien (18) peut être réglée autour de six degrés de liberté à l'aide du premier bras (12).

7. Dispositif (10) selon l'une quelconque des revendications précédentes, **caractérisé en ce que** l'unité de support (16) comprend un rail et **en ce que** le second bras (12) a une coulisse, qui est supportée par le rail et est mobile dans le sens longitudinal du rail à l'aide de l'unité actionneur (56),
dans lequel, de préférence, l'unité actionneur (56) comprend un premier moteur servant à déplacer la coulisse.

8. Dispositif (10) selon l'une quelconque des revendications précédentes, **caractérisé en ce qu'**un moteur servant à déplacer les articulations (32 à 36) est associé à chaque articulation (32 à 36) du second bras (14).

9. Dispositif (10) selon la revendication 7, **caractérisé en ce que** le second bras (14) a deux plaques de raccordement (42, 44), dans lequel la première articulation (32) est disposée sur la coulisse, la première plaque de raccordement (42) entre la première et la deuxième articulations (32, 34), et la seconde plaque de raccordement (44) entre la deuxième et la troisième articulations (34, 36).

10. Dispositif (10) selon la revendication 2, **caractérisé en ce que** la seconde unité de maintien (20) est disposée sur la troisième articulation (36).

11. Dispositif (10) selon l'une quelconque des revendications précédentes, **caractérisé en ce que** la première articulation (32) a un premier axe de rotation (38) et la deuxième articulation (34) a un second axe de rotation (40) et **en ce que** les premier et second axes de rotation (38, 40) sont parallèles l'un par rapport à l'autre, ou
**en ce que** la troisième articulation (36) a trois axes de rotation (50 à 54) disposés perpendiculairement les uns par rapport aux autres.

12. Dispositif (10) selon l'une quelconque des revendications précédentes, **caractérisé en ce que** l'unité d'opération (60) comprend un joystick.

13. Dispositif (10) selon l'une quelconque des revendications précédentes, **caractérisé en ce que** l'unité d'opération (60) a six degrés de liberté, et/ou en ce que sur l'unité de support (16) un coussin de cuisse (64) et/ou un appuie-jambe (62) est/sont fixé(s) de manière amovible.

14. Table d'opération (100), **caractérisée en ce que** sur la table d'opération (100) au moins un dispositif (10) selon l'une quelconque des revendications 1 à 13 est fixé de manière amovible .

15. Table d'opération (100) selon la revendication 14, **caractérisée en ce que** le dispositif (10) est fixé au moyen d'une fixation de type friction.
